(19) Europäisches Patentamt
European Patent Office
Office européen des brevets

(11) Veröffentlichungsnummer: **0 010 250**
**B1**

(12) # EUROPÄISCHE PATENTSCHRIFT

(45) Veröffentlichungstag der Patentschrift: **08.02.84**

(21) Anmeldenummer: **79103861.5**

(22) Anmeldetag: **08.10.79**

(51) Int. Cl.[3]: **A 01 N 41/06,**
A 01 N 41/10,
C 07 C 143/74,
C 07 C 147/12,
C 07 C 147/14,
C 07 C 149/425

(54) **Verwendung von Sulfonaniliden zur Hemmung des Pflanzenwachstums.**

(30) Priorität: **23.10.78 DE 2845997**

(43) Veröffentlichungstag der Anmeldung:
**30.04.80 Patentblatt 80/9**

(45) Bekanntmachung des Hinweises auf die Patenterteilung:
**08.02.84 Patentblatt 84/6**

(84) Benannte Vertragsstaaten:
**BE CH DE FR GB IT NL**

(56) Entgegenhaltungen:
**DE - A - 2 018 969**
**FR - A - 2 287 171**
**FR - A - 2 291 190**
**FR - A - 2 378 754**
**NL - A - 7 803 725**
**US - A - 3 246 974**
**US - A - 3 948 987**

**Die Akte enthält technische Angaben, die nach dem Eingang der Anmeldung eingereicht wurden und die nicht in dieser Patentschrift enthalten sind.**

(73) Patentinhaber: **BAYER AG**
**Zentralbereich Patente, Marken und Lizenzen**
**D-5090 Leverkusen 1, Bayerwerk (DE)**

(72) Erfinder: **Kühle, Engelbert, Dr.**
**von-Bodelschwingh-Strasse 42**
**D-5060 Bergisch-Gladbach 2 (DE)**
Erfinder: **Lürssen, Klaus, Dr.**
**August-Kierspel-Strasse 89**
**D-5060 Bergisch-Gladbach 2 (DE)**

**EP 0 010 250 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Verwendung von Sulfonaniliden zur Hemmung des Pflanzenwachstums

Die vorliegende Erfindung betrifft die Verwendung von teilweise bekannten Sulfonaniliden als Wirkstoffe zur Hemmung des Pflanzenwachstums.

Es ist bereits bekannt geworden, daß 2-Chlorethylphosphonsäure pflanzenwuchsregulierende Eigenschaften aufweist (vgl. Deutsche Offenlegungsschrift 2 050 245). Die Wirksamkeit dieses Stoffes ist jedoch, — vor allem bei niedrigen Aufwandmengen —, nicht immer ganz befriedigend.

Weiterhin sind aus der DE—A 2 018 969 und der FR—A 2 378 754 zahlreiche Halogenmethylsulfonanilide mit herbiziden und pflanzenwuchsregulierenden Eigenschaften bekannt. Es werden jedoch keine derartigen Stoffe bechrieben, in denen die Sulfonanilid-Gruppe keinen Halogenmethyl-Rest trägt.

Darüberhinaus wurden in der FR—A 2 291 190, der NL—A 7 803 725 und der US—A 3 948 987 herbizid und pflanzenwuchsregulierend wirksame (2-Trifluormethyl)-alkylsulfonanilide offenbart, die in der para-Stellung zur Alkylsulfonyl-Gruppe zwingend durch Alkylthio, Alkylsulfinyl oder Alkylsulfonyl bzw. durch Phenylthio, Phenylsulfinyl oder Phenylsulfonyl substituiert sind. Entsprechende Verbindungen, die in der para-Position andere als die gegenanten Reste aufweisen, werden hingegen nicht erwähnt. Im übrigen läßt die pflanzenwuchshemmende Wirkung dieser Stoffe in manchen Fällen zu wünschen übrig.

Gemäß FR—A 2 287 171 lassen sich auch bestimmte Benzolsulfonamide, in denen die Sulfonyl-Gruppe außer mit dem Stickstoffatom noch mit einem Phenylrest verbunden ist, als Herbizide und Pflanzenwuchsregulatoren verwenden. Die allgemeine Formel umfaßt aber keine Stoffe, in denen statt des Phenylrestes ein Alkylsubstituent oder eine Dialkylamino-Gruppe vorhanden ist.

Schließlich betrifft die US—A 3 246 974 Sulfonamide mit herbiziden Eigenschaften. Ein Einsatz dieser Verbindungen zur Hemmung des Wachstums von Kulturpflanzen ist jedoch nicht bekannt.

Es wurde nun gefunden, daß die Sulfonanilide der Formel

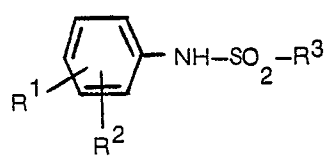

(I)

in welcher

$R^1$ für gegebenenfalls durch Halogen substituiertes Phenoxy, Alkylthio mit 1 bis 4 Kohlenstoffatomen, Alkylsulfonylamino-alkylthio mit 1 bis 4 Kohlenstoffatomen in der Alkylsulfonylgruppe und 1 bis 4 Kohlenstoffatomen in der Alkylthiogruppe oder für die Gruppierung der Formel $—S(O)_nR^6$ steht, in welcher

$R^6$ für Alkyl mit 1 bis 4 Kohlenstoffatomen oder Halogenalkyl mit bis zu 4 Kohlenstoffatomen und bis zu 5 Halogenatomen steht und

n für 1 oder 2 steht,

$R^2$ für Wasserstoff, Chlor oder Halogenalkyl mit bis zu 4 Kohlenstoffatomen und bis zu 5 Halogenatomen steht und

$R^3$ für die Gruppierung der Formel

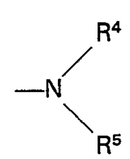

steht,

worin

$R^4$ und $R^5$ unabhängig voneinander für Alkyl mit 1 bis 4 Kohlenstoffatomen stehen,

oder

$R^3$ für Alkyl mit 1 bis 4 Kohlenstoffatomen steht, sofern

$R^1$ nicht für Alkylthio mit 1 bis 4 Kohlenstoffatomen steht und

$R^6$ nicht für Alkyl mit 1 bis 4 Kohlenstoffatomen steht,

sehr gut zur Hemmung des Pflanzenwachstums geeignet sind.

Überraschenderweise zeigen die erfindungsgemäß verwendbaren Sulfonanilide der Formel (I) eine wesentlich bessere pflanzenwuchshemmende Wirksamkeit als die aus dem Stand der Technik bekannte 2-Chlorethylphosphonsäure, welche ein hochaktiver Wirkstoff gleicher Wirkungsart ist. Außerdem übertreffen die erfindungsgemäß verwendbaren Sulfonanilide der Formel (I) im Gegensatz zu den Erwartungen auch die konstitutionell ähnlichsten, aus der FR—A 2 291 190 und der US—A 3 948 987 vorbekannten Stoffe bezüglich ihrer pflanzenwuchshemmenden Wirksamkeit. Die

erfindungsgemäß verwendbaren Verbindungen der Formel (I) stellen somit eine wertvolle Bereicherung der Technik dar.

Als Beispiele für erfindungsgemäß verwendbare Sulfonanilide seien im einzelnen die folgenden Verbindungen der Formel (I) genannt:

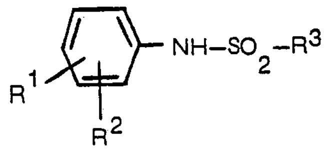

(I)

| $R^3$ | $R^1$ | $R^2$ |
|---|---|---|
| 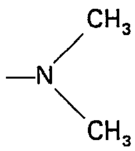 | $4—SO_2CH_3$ | 3-Cl |
| 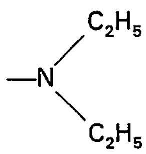 | $4—SO_2CH_3$ | 3-Cl |
| $—N(C_3H_7)_2$ | $4—SO_2CH_3$ | 3-Cl |
| $—CH_3$ | 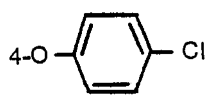 | 3-Cl |
| $—C_2H_5$ | $2—S—CH_2—CH_2—NH—SO_2CH_3$ | H |
| $—CH_3$ | $2—S—CH_2—CH_2—NH—SO_2—C_2H_5$ | H |

Die erfindungsgemäß verwendbaren Sulfonanilide der Formel (I) sind im Prinzip bekannt (vgl. DE—A 2 703 477 und DE—A 2 551 027). Sie lassen sich nach mehreren Verfahren herstellen. So erhält man

a) Sulfonanilide der Formel (I), indem Aniline der Formel

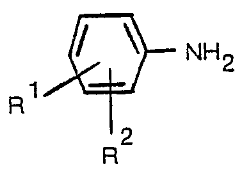

(II)

in welcher

$R^1$ und $R^2$ die oben angegebene Bedeutung haben,

mit Sulfochloriden der Formel

$$Cl—SO_2—R^3 \qquad (III)$$

in welcher

$R^3$ die oben angegebene Bedeutung hat,

in Gegenwart eines Säureakzeptors sowie gegebenenfalls in Gegenwart eines Verdünnungsmittels umsetzt, oder

b) diejenigen Sulfonanilide der Formel (I), in denen $R^1$ für die Gruppierung der Formel $—S(O)_nR^6$ steht, worin $R^6$ und n die oben angegebene Bedeutung haben, indem man Sulfonanilide der Formel

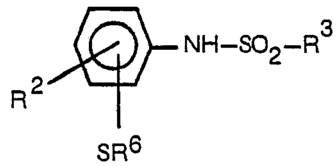

(Ia)

in welcher

$R^3$, $R^2$ und $R^6$ die oben angegebene Bedeutung haben,

gegebenenfalls in Gegenwart eines Verdünnungsmittels oxidiert.

Die bei dem Verfahren (a) als Ausgangsstoffe benötigten Aniline der Formel (II) sind bekannt oder lassen sich nach im Prinzip bekannten Verfahren herstellen (vgl. z.B. Houben-Weyl, "Methoden der organischen Chemie", Band IX, Seite 105 ff., 4. Auflage 1955 sowie DE—A 2 551 027).

Man erhält Aniline der Formel (II) z.B. indem man

$\alpha$) Nitroverbindungen der Formel

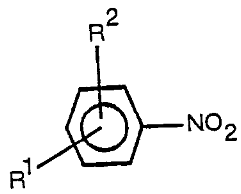

(IV)

in welcher

$R^1$ und $R^2$ die oben angegebene Bedeutung haben,

mit Wasserstoff in Gegenwart eines Katalysators, wie Raney-Nickel oder Cobaltsulfid, sowie in Gegenwart eines Verdünnungsmittels, wie z.B. Methanol, bei Temperaturen zwischen 20°C und 150°C reduziert,

oder indem man

$\beta$) Amino-thiophenole der Formel

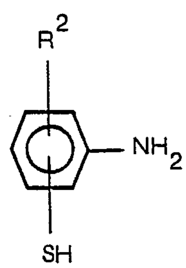

(V)

in welcher

$R^2$ die oben angegebene Bedeutung hat,

mit Halogenverbindungen der Formel

$R^6$-Hal (VI)

in welcher

$R^6$ die oben angegebene Bedeutung hat und

Hal für Chlor oder Brom steht,

in Gegenwart eines Verdünnungsmittels, wie Methylenchlorid, sowie gegebenenfalls in Gegenwart eines Säurebindemittels, wie Pyridin bei Temperaturen zwischen 0°C und 120°C, umsetzt, und gegebenenfalls anschließend oxidiert,

oder indem man

$\gamma$) Isocyanate der Formel

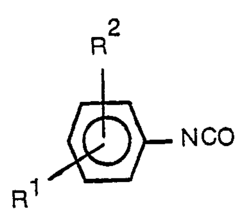

(VII)

in welcher

$R^1$ und $R^2$ die oben angegebene Bedeutung haben,

mit starken Säuren, wie z.B. Schwefelsäure, in Gegenwart eines Verdünnungsmittels, wie z.B, Chlorbenzol, bei Temperaturen zwischen 20°C und 100°C behandelt.

Die Verbindungen der Formeln (III) bis (VII) sind bekannt oder lassen sich nach im Prinzip bekannten Verfahren herstellen.

Die Sulfonanilide der Formel (Ia) lassen sich nach dem Verfahren (a) in einfacher Weise herstellen.

Als Oxidationsmittel kommen bei der Durchführung des Verfahrens (b) alle üblichen sauerstoffabgebenden Oxidationsmittel in Frage. Hierzu gehören vorzugsweise Wasserstoffperoxid, Peressigsäure und m-Chlorperbenzoesäure.

Bei der Durchführung des Verfahrens (a) können als Säureakzeptoren alle üblichen Säurebindemittel fungieren. Hierzu gehören vorzugsweise Alkalihydroxide wie Natriumhydroxid und Kaliumhydroxid, ferner Alkalicarbonate wie Natriumcarbonat und Kaliumcarbonat, außerdem Alkalialkoholate wie Natriummethylat und Natriumethylat, weiterhin aliphatische, aromatische oder heterocyclische Amine wie beispielsweise Triethylamin, Dimethylamin, Dimethylanilin, Dimethylbenzylamin und Pyridin.

Als Verdünnungsmittel können bei der Umsetzung nach dem Verfahren (a) alle inerten organischen Lösungsmittel verwendet werden. Hierzu gehören vorzugsweise aliphatische oder aromatische Kohlenwasserstoffe, wie Petrolether, Benzin, Benzol, Toluol und Xylol, ferner chlorierte aliphatische oder aromatische Kohlenwasserstoffe, wie Methylenchlorid, Chloroform, Tetrachlorkohlenstoff und Chlorbenzol, weiterhin Ether, wie Diethylether, Dibutylether, Tetrahydrofuran und Dioxan, und außerdem Ketone, wie Aceton. Darüber hinaus kann auch Wasser als Verdünnungsmittel eingesetzt werden.

Die Reaktionstemperaturen können bei dem Verfahren (a) innerhalb eines größeren Bereiches variiert werden. Im allgemeinen arbeitet man bei Temperaturen zwischen 0°C und 100°C, vorzugsweise zwischen 10°C und 50°C.

Bei der Durchführung des Verfahrens (a) setzt man auf 1 Mol Anilin der Formel (II) 1 bis 3 Mol an Sulfochlorid der Formel (III) ein. Bei Verwendung von Sulfochlrid in mehr als der äquimolaren Menge kann auch das zweite Wasserstoffatom am Stickstoff des Anilins durch den Rest $—SO_2R^3$ substituiert werden. Letztere Verbindungen lassen sich jedoch durch Behandlung mit starken Basen, wie z.B. Kaliumhydroxid, in das gewünschte Produkt überführen. Die Isolierung der Reaktionsprodukte erfolgt nach üblichen Methoden. Im allgemeinen geht man so vor, daß man das Reaktionsgemisch mit einer verdünnten starken Säure versetzt. Dabei scheidet sich das Reaktionsprodukt in vielen Fällen kristallin ab. Ist im Reaktionsprodukt ein größerer Anteil an solchen Substanzen vorhanden, in denen das Stickstoffatom des eingesetzten Anilins durch 2 $—SO_2R^3$-Gruppen substituiert ist, so empfiehlt es sich, nach dem Versetzen mit verdünnter Säure anschließend mit einem mit Wasser nicht mischbaren organischen Lösungsmittel zu extrahieren, dann das Lösungsmittel zu entfernen, den verbleibenden Rückstand mit einer starken Base zu behandeln und anschließend anzusäuern.

Bei der Durchführung des Verfahrens (b) können als Verdünnungsmittel alle üblicherweise für derartige Oxidationen verwendbaren inerten organischen Lösungsmittel eingesetzt werden. Hierzu gehören vorzugsweise Eisessig und Methylenchlorid.

Die Reaktionstemperaturen können bei der Oxidation gemäß Verfahren (b) innerhalb eines größeren Bereiches variiert werden. Im allgemeinen arbeitet man bei Temperaturen zwischen 0°C und 120°C, vorzugsweise zwischen 50°C und 100°C.

Bei der Durchführung des Verfahrens (b) setzt man auf 1 Mol an Sulfonanilid der Formel (Ia) eine äquimolare Menge oder einen Überschuß an Oxidationsmittel ein, und zwar in Abhängigkeit davon, ob die Sulfinyl- oder die Sulfonyl-Verbindung der Formel (I) synthetisiert werden soll. Die Aufarbeitung erfolgt nach üblichen Methoden. Im allgemeinen verfährt man in der Weise, daß man nach beendeter Umsetzung mit Wasser versetzt. Dabei scheidet sich das Reaktionsprodukt häufig unmittelbar in kristalliner Form ab. Ist dies nicht der Fall, so extrahiert man das Reaktionsgemisch mit einem mit Wasser nicht mischbaren organischen Lösungsmittel, trennt die Phasen und entfernt das Lösungsmittel.

Die erfindungsgemäß verwendbaren Wirkstoffe greifen in den Metabolismus der Pflanzen ein und können zur Hemmung des Wachstums von Kulturpflanzen eingesetzt werden.

Sie können zum Beispiel zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park- und Sportanlagen, an Straßenrändern, auf Flughäfen oder in Obstanlagen reduziert werden. Von Bedeutung ist auch die Hemmung des Wuchses von krautigen und holzigen Pflanzen an Straßenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens ("Lagerns") der Pflanzen vor der Ernte verringert oder vollkommen beseitigt. Außerdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne daß die Gefahr besteht, daß das Getreide lagert.

Eine Hemmung des vegetativen Wachstums ermöglicht bei vielen Kulturpflanzen eine dichtere Anpflanzung, sodaß Mehrerträge bezogen auf die Bodenfläche erzielt werden können. Ein Vorteil der so erzielten kleineren Pflanzen ist auch, daß die Kultur leichter bearbeitet und beerntet werden kann.

Eine Hemmung des vegetativen Wachstums der Pflanzen kann auch dadurch zu Ertragssteigerungen führen, daß die Nährstoffe und Assimilate in stärkerem Maße der Blüten- und Fruchtbildung zugute kommen als den vegetativen Pflanzenteilen.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, sowie ULV-Formulierungen.

Diese Formulierungen werden in an sich bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Äther, z.B. Alkylarylpolyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol- und Metallphthalkocyananinfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in den Formulierungen in Mischung mit anderen bekannten Wirkstoffen vorliegen, wie Fungizide, Insektizide; Akarizide und Herbizide, sowie in Mischungen mit Düngemitteln und anderen Wachstumsregulatoren.

Die Wirkstoffe können als solche, in Form ihrer Formulierungen oder der daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Begasen usw. Es ist ferner möglich, die Wirkstoffe nach dem Ultra-Low-Volume-Verfahren auszubringen, Pflanzen oder Pflanzenteile mit der Wirkstoffzubereitung oder dem Wirkstoff selbst zu bestreichen oder die Wirkstoffzubereitung oder den Wirkstoff selbst in den Boden zu injizieren. Es kann auch das Saatgut der Pflanze behandelt werden.

Die Wirkstoffkonzentrationen können in einem größeren Bereich variiert werden. Im allgemeinen verwendet man pro Hektar Bodenfläche 0,01 bis 50 kg, bevorzugt 0,05 bis 10 kg an Wirkstoff.

Für die Anwendungszeit gilt, daß die Anwendung der Wuchshemmer in einem bevorzugten Zeitraum vorgenommen wird, dessen genaue Abgrenzung sich nach den klimatischen und vegetativen Gegebenheiten richtet.

Im folgenden wird die Aktivität der erfindungsgemäß verwendbaren Sulfonanilide der Formel (I) als Pflanzenwuchshemmer durch einige Beispiele illustriert.

Beispiel A

*Wuchshemmung bei Gehölzen* (Acer pesudoplatanus)

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Einjährige Sämlinge mit einer Wuchshöhe von 25 cm werden mit den Wirkstoffzubereitungen tropfnaß besprüht. Nach 6 Wochen Wachstum im Gewächshaus wird der Zuwachs gemessen und die Wuchshemmung in Prozent des Zuwachses der Kontrollpflanzen berechnet. 100% bedeuten den Stillstand des Wachstums und 0% ein Wachstum entsprechend dem der Kontrollpflanzen.

Wirkstoffe, Wirkstoffkonzentrationen und Resultate gehen aus der nachfolgenden Tabelle hervor.

TABELLE A

Wuchshemmung bei Gehölzen (Acer pseudoplatanus)

| Wirkstoff | Wirkstoff-konzentration in % | Wuchshemmung in % |
|---|---|---|
| – (Kontrolle) | – | 0 |
| 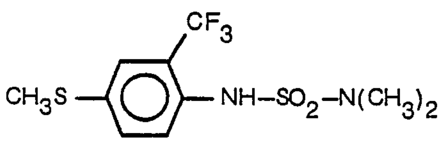 | 0,4 | 41 |

Beispiel B

*Wuchshemmung bei Gehölzen* (Alnus glutinosa)

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Einjährige Sämlinge mit einer Wuchshöhe von 25 cm werden mit den Wirkstoffzubereitungen tropfnaß besprüht. Nach 6 Wochen Wachstum im Gewächshaus wird der Zuwachs gemessen und die Wuchshemmung in Prozent des Zuwachses der Kontrollpflanzen berechnet. 100% bedeuten den Stillstand des Wachstums und 0% ein Wachstum entsprechend dem der Kontrollpflanzen.

Wirkstoffe, Wirkstoffkonzentrationen und Resultate gehen aus der nachfolgenden Tabelle hervor.

TABELLE B

Wuchshemmung bei Gehölzen (Alnus glutinosa)

| Wirkstoff | Wirkstoff-konzentration in % | Wuchshemmung in % |
|---|---|---|
| 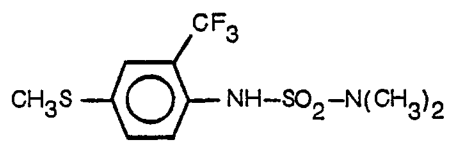 | 0,4 | 87 |
| 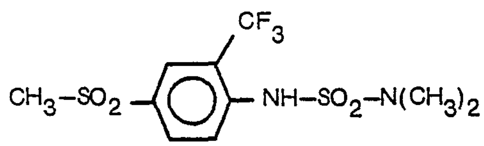 | 0,4 | 61 |
| – (Kontrolle) | – | 0 |

Beispiel C

*Wuchshemmung bei Weizen*

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Weizenpflanzen werden im Gewächshaus bis zum 2-Blatt-Stadium angezogen. In diesem Stadium werden die Pflanzen tropfnaß mit den Wirkstoffzubereitungen besprüht. Nach 3 Wochen wird bei allen Pflanzen der Zuwachs gemessen und die Wuchshemmung in % des Zuwachses der Kontrollpflanzen berechnet. Es bedeuten 100% den Stillstand des Wachstums und 0% ein Wachstum entsprechend dem der Kontrollpflanzen.

Wirkstoffe, Wirkstoffkonzentrationen und Resultate gehen aus der nachfolgenden Tabelle hervor.

TABELLE C

Wuchshemmung bei Weizen

| Wirkstoff | Wirkstoff-konzentration in % | Wuchshemmung in % |
|---|---|---|
| – (Kontrolle) | – | 0 |
| 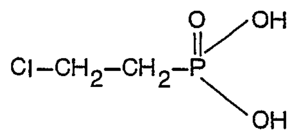 (bekannt) | 0,05 | 0 |
| 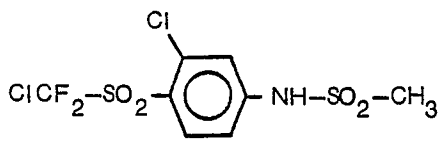 | 0,05 | 70 *) |
| 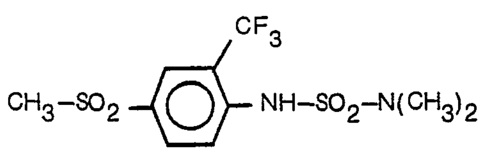 | 0,05 | 70 |

*) dunkelgrüne Blattfarbe

Beispiel D

*Wuchshemmung bei Gras (Festuca pratensis)*

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Gras (Festuca pratensis) wird im Gewächshaus bis zu einer Wuchshöhe vor 5 cm angezogen. In diesem Stadium werden die Pflanzen mit den Wirkstoffzubereitungen tropfnaß besprüht. Nach 3 Wochen wird der Zuwachs gemessen und die Wuchshemmung in Prozent des Zuwachses der Kontrollpflanzen berechnet. Es bedeuten 100% den Stillstand des Wachstums und 0% ein Wachstum entsprechend dem der Kontrollpflanzen.

Wirkstoffe, Wirkstoffkonzentrationen und Resultate gehen aus der nachfolgenden Tabelle hervor.

TABELLE D

Wuchshemmung bei Gras (Festuca pratensis)

| Wirkstoff | Wirkstoff-konzentration in % | Wuchshemmung in % |
|---|---|---|
| – (Kontrolle) | – | 0 |
| 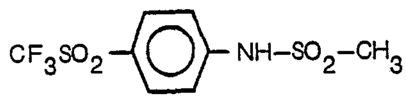 | 0,05 | 90 |
| 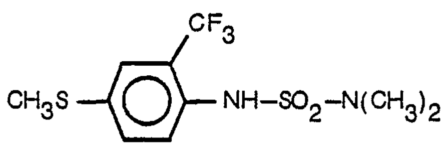 | 0,05 | 100 |
| 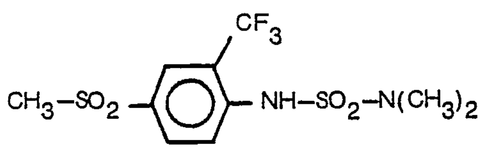 | 0,05 | 100 |

Beispiel E

*Wuchshemmung bei Baumwolle*

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 3 Wochen wird der Zuwachs der Pflanzen gemessen und die Wuchshemmung in Prozent des Zuwachses der Kontrollpflanzen berechnet. Es bedeuten 100% den Stillstand des Wachstums und 0% ein Wachstum entsprechend dem der Kontrollpflanzen.

Wirkstoffe, Wirkstoffkonzentrationen und Resultate gehen aus der nachfolgenden Tabelle hervor.

TABELLE E

Wuchshemmung bei Baumwolle

| Wirkstoff | Wirkstoff-konzentration in % | Wuchshemmung in % |
|---|---|---|
| – (Kontrolle) | | 0 |
| 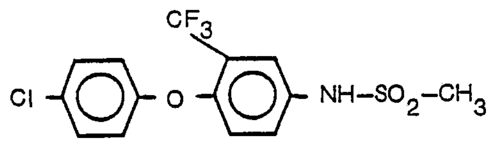 | 0,05 | 45 |
| 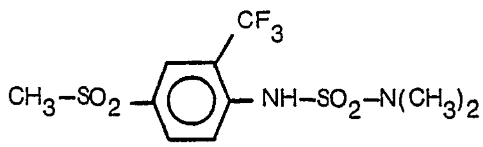 | 0,05 | 50 |
| 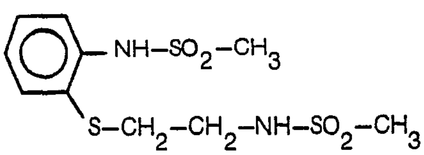 | 0,05 | 85 |

Beispiel F

*Wuchshemmung bei Sojabohnen*

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Sojabohnenpflanzen werden im Gewächshaus bis zur vollen Entfaltung des ersten Folgeblattes angezogen. In diesem Stadium werden die Pflanzen tropfnaß mit den Wirkstoffzubereitungen besprüht. Nach 3 Wochen wird bei allen Pflanzen der Zuwachs gemessen und die Wuchshemmung in Prozent des Zuwachses der Kontrollpflanzen berechnet. Es bedeuten 100% den Stillstand des Wachstums und 0% ein Wachstum entsprechend dem der Kontrollpflanzen.

Wirkstoffe, Wirkstoffkonzentrationen und Resultate gehen aus der nachfolgenden Tabelle hervor.

TABELLE F

Wuchshemmung bei Sojabohnen

| Wirkstoff | Wirkstoff-konzentration in % | Wuchshemmung in % |
|---|---|---|
| – (Kontrolle) | – | 0 |
| 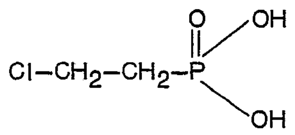 (bekannt) | 0,05 | 50 |
| 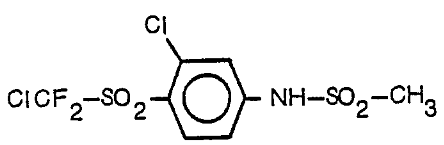 | 0,05 | 65 |
| 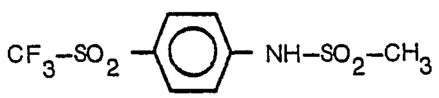 | 0,05 | 75 |
| 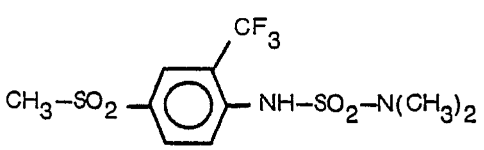 | 0,05 | 100 |

Beispiel G

*Wuchshemmung bei Gerste*

Lösungsmittel: 30 Gewichtsteile Dimethylformamid

Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Gerstepflanzen werden im Gewächshaus bis zum 2-Blattstadium angezogen. In diesem Stadium werden die Pflanzen tropfnaß-mit den Wirkstoffzubereitungen besprüht. Nach 3 Wochen wird bei allen Pflanzen der Zuwachs gemessen und die Wuchshemmung in Prozent des Zuwachses der Kontrollpflanzen berechnet. Es bedeuten 100% den Stillstand des Wachstums und 0% ein Wachstum entsprechend dem der Kontrollpflanzen.

Wirkstoffe, Wirkstoffkonzentrationen und Resultate gehen aus der nachfolgenden Tabelle hervor.

TABELLE G

Wuchshemmung bei Gerste

| Wirkstoff | Wirkstoff-konzentration in % | Wuchshemmung in % |
|---|---|---|
| – (Kontrolle) | – | 0 |
| 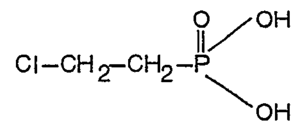 (bekannt) | 0,05 | 0 |
| 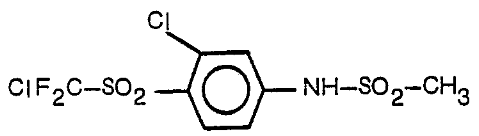 | 0,05 | 90*) |
| 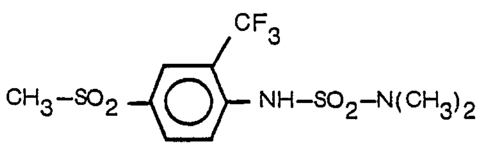 | 0,05 | 100 |

*) dunkelgrüne Blattfarbe

## Herstellungsbeispiele

### Beispiel 1

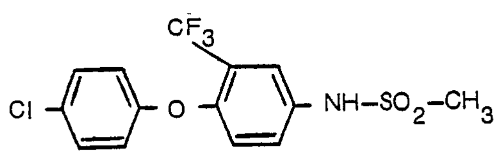

Eine Lösung von 20,1 g (70 m Mol) 2-Trifluormethyl-4-amino-4'-chlor-diphenyl-ether in 100 ml Pyridin wird bei 0°C bis 5°C unter Rühren tropfenweise mit 8,5 g (74 mMol) Methansulfochlorid versetzt. Man rührt noch etwa 30 Minuten bei Raumtemperatur, gießt dann das Reaktionsgemisch in verdünnte wäßrige Salzsäure und saugt den ausfallenden Feststoff ab. Dieses Produkt wird in verdünnter wäßriger Natronlauge gelöst. Man behandelt die Lösung mit Aktivkohle filtriert und säuert mit verdünnter wäßriger Salzsäure an. Dabei fällt das Reaktionsprodukt in kristalliner Form aus. Man erhält auf diese Weise 3-Trifluormethyl-4-(4-chlorphenoxy)-methylsulfonanilid in Form einer Festsubstanz vom Schmelzpunkt 125—128°C.

In analoger Weise werden die in der nachfolgenden Tabelle 1 formelmäßig aufgeführten Sulfonanilide der Formel (I) hergestellt.

TABELLE 1

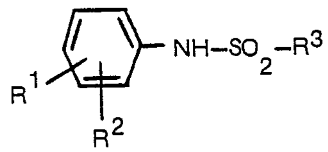

(I)

| Beispiel Nr. | $R^3$ | $R^1$ | $R^2$ | Schmelzpunkt [°C] bzw. Brechungsindex |
|---|---|---|---|---|
| 2 | $-N(CH_3)_2$ | $4\text{-}SCH_3$ | $2\text{-}CF_3$ | $n_D^{20}$ = 1,5399 |
| 3 | $-CH_3$ | $4\text{-}SO_2CF_2Cl$ | 3-Cl | 163 – 165 |
| 4 | $-CH_3$ | $4\text{-}SO_2CF_3$ | H | 163 – 165 |

Beispiel 5

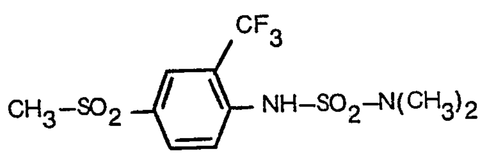

Durch Oxidation der im Beispiel 2 beschriebenen Verbindung mit Wasserstoffperoxid in Eisessig wurde die Verbindung der oben angegebenen Formel als Festsubstanz vom Schmelzpunkt 122—123°C erhalten.

Beispiel 6

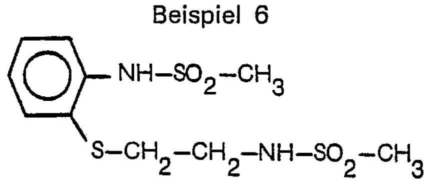

17 g 2-($\beta$-Aminoethylmercapto)-anilin werden in 100 ml Pyridin gelöst und bei 0—5°C mit 23 g Methansulfochlorid versetzt. Man rührt noch eine Weile nach und gießt dann das Reaktionsgemisch in verdünnte wäßrige Salzsäure. Das dabei anfallende Produkt wird in Methylenchlorid aufgenommen. Man trocknet und engt ein. Auf diese Weise erhält man 25 g (28% der Theorie) des gewünschten Reaktionsproduktes in Form eines zähen Öles. Die Struktur der Substanz wurde mit Hilfe des Kernresonanz-Spektrums bestimmt.

Beispiel (II-1)

a)

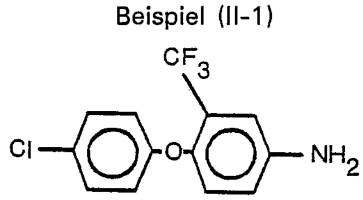

185 g 2-Trifluormethyl-4-nitro-4'-chlordiphenylether werden in 900 ml Methanol gelöst und nach Zusatz von 30 g Raney-Nickel in einem Autoklaven bei 50°C unter einem Wasserstoffdruck von 55 bar hydriert. Nach dem Abkühlen und Entspannen wird das Reaktionsgemisch filtriert und nach dem Abziehen des Lösungsmittels destilliert. Man erhält 2-Trifluormethyl-4-amino-4'-chlor-diphenylether.

$Kp_{0,1}$ 145—147°C
$n_D^{20}$ = 1,5650

b)

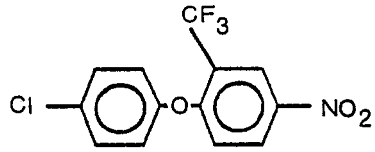

1 Mol 4-Chlorphenol und 56 g Kaliumhydroxid werden in 600 ml Dimethylsulfoxid vorgelegt und unter Rühren auf 130°C erwärmt. Bei dieser Temperatur werden 226 g (1 Mol)5-Nitro-2-chlor-benzotrifluorid zugegeben. Anschließend wird der Ansatz 2 Stunden bei 145°C gerührt. Danach wird das Lösungsmittel unter vermindertem Druck abgezogen und der Rückstand auf Wasser gegeben. Die organische Phase wird abgetrennt. Der wäßrige Rückstand wird mit Methylenchlorid extrahiert. Man vereinigt die organischen Phasen, wäscht zweimal mit Wasser, trocknet über Natriumsulfat und destilliert. Nach einem Vorlauf von unverändertem Ausgangsmaterial erhält man 2-Trifluormethyl-4-nitro-4'-chlor-diphenylether in Form einer Flüssigkeit.

Beispiel (II-2)

a)

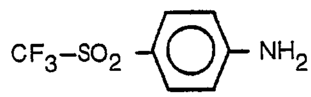

4-Nitrophenyl-trifluormethylsulfon wird in Methanol gelöst und in einem Autoklaven in Gegenwart von Raney-Nickel als Katalysator bei 50°C unter einem Wasserstoff-Druck von 50 bar hydriert. Die Aufarbeitung erfolgt in der Weise, daß man das Reaktionsgemisch filtriert und dann einengt. Man erhält 4-Trifluormethylsulfon-anilin in 95 %iger Ausbeute in Form von Kristallen mit einem Schmelzpunkt von 94—95°C.

b)

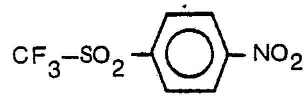

Eine Lösung von 53 g 4-Trifluormethylmercaptonitrobenzol in 100 ml Essigsäure wird bei Raumtemperatur unter Rühren in ein Gemisch aus 66 g Chromtrixoid in 100 ml Essigsäure getropft. Nach Ende der Zugabe erwärmt man auf 90°C und rührt bei dieser Temperatur noch 1 Stunde nach. Anschließend gibt man das Reaktionsgemisch auf Eis, extrahiert mit Methylenchlorid und zieht das Lösungsmittel ab. Man erhält 35 g 4-Nitrophenyl-trifluormethylsulfon vom Schmelzpunkt 83—84°C.

Beispiel (II-3)

a)

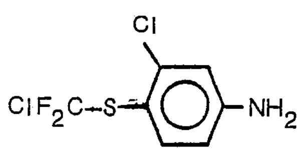

Eine Lösung von 200 g 3-Chlor-4-difluorchlormethylmercapto-phenylisocyanat in 200 ml Chlorbenzol wird bei 12—13°C unter intensivem Rühren in 320 ml konzentrierte Schwefelsäure getropft. Anschließend wird 30 Minuten bis zu Beendigung der Kohlendioxid-Entwicklung nachgerührt. Danach gießt man das Reaktionsgemisch auf Eis, saugt den kristallinen Niederschlag ab, versetzt ihn mit wäßriger Natronlauge und nimmt mit Toluol auf. Man destilliert das Toluol ab und erhält 150 g eines Rückstandes mit einem Brechungsindex $n_D^{20}$: 1,5927. Gemäß Gaschromatogramm beträgt der Gehalt an 3-Chlor-4-difluorchlormethylmercapto-anilin 97,2%.

Die Reinigung des Produktes erfolgt durch Destillation.

$Kp_{0,3}$: 120°C
$n_D^{20}$: 1,5925

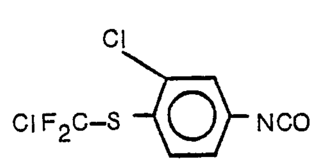

1880 g 3-Chlor-4-trichlormethylmercapto-phenylisocyanat werden bei 5—10°C innerhalb von 2

Stunden in 840 ml wasserfreie Flußsäure eingetropft. Dabei setzt eine heftige Chlorwasserstoff-Entwicklung ein. Nach beendeter Zugabe läßt man das Gemisch bei 20°C noch weitere 8 Stunden reagieren bis sich kein Chlorwasserstoff mehr entwickelt. Danach wird die überschüssige. Flußsäure abgezogen, und der Rückstand wird destilliert. Man erhält 1225 g an 3-Chlor-4-difluorchlormethylmercaptophenylisocyanat.

$Kp_{14}$: 139°C
$n_D^{20}$: 1,5650.

**Patentanspruch**

Verwendung von Sulfonaniliden der Formel

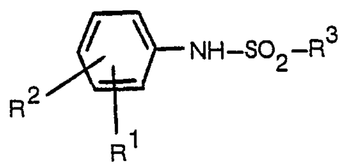

(I)

in welcher

$R^1$ für gegebenenfalls durch Halogen substituiertes Phenoxy, Alkylthio mit 1 bis 4 Kohlenstoffatomen, Alkylsulfonylamino-alkylthio mit 1 bis 4 Kohlenstoffatomen in der Alkylsulfonylgruppe und 1 bis 4 Kohlenstoffatomen in der Alkylthiogruppe oder für die Gruppierung der Formel $—S(O)_nR^6$ steht,

in welcher

$R^6$ für Alkyl mit 1 bis 4 Kohlenstoffatomen oder Halogenalkyl mit 1 bis 4 Kohlenstoffatomen und bis zu 5 Halogenatomen steht

und

n für 1 oder 2 steht,

$R^2$ für Wasserstoff, Chlor oder Halogenalkyl mit 1 bis 4 Kohlenstoffatomen und bis zu 5 Halogenatomen steht und

$R^3$ für die Gruppierung der Formel

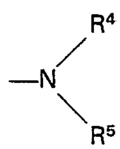

steht,

worin

$R^4$ und $R^5$ unabhängig voneinander für Alkyl mit 1 bis 4 Kohlenstoffatomen stehen,

oder

$R^3$ für Alkyl mit 1 bis 4 Kohlenstoffatomen steht, sofern

$R^1$ nicht für Alkylthio mit 1 bis 4 Kohlenstoffatomen steht und

$R^6$ nicht für Alkyl mit 1 bis 4 Kohlenstoffatomen steht,

zur Hemmung des Pflanzenwachstums.

**Claim**

Use of sulphonanilides of the formula

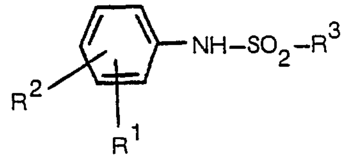

(I)

in which

$R^1$ represents optionally halogen-substituted phenoxy, alkylthio with 1 to 4 carbon atoms, alkylsulphonylaminoalkylthio with 1 to 4 carbon atoms in the alkylsulphonyl group and 1 to 4 carbon atoms in the alkylthio group or the grouping of the formula $—S(O)_nR^6$, in which

$R^6$ represents alkyl with 1 to 4 carbon atoms or halogenoalkyl with 1 to 4 carbon atoms and up to 5 halogen atoms, and

n represents 1 or 2,

$R^2$ represents hydrogen, chlorine or halogenalkyl with 1 to 4 carbon atoms and up to 5 halogen atoms and

$R^3$ represents the grouping of the formula

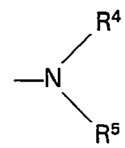

wherein

$R^4$ and $R^5$ independently of one another represent alkyl with 1 to 4 carbon atoms,

or

$R^3$ represents alkyl with 1 to 4 carbon atoms, insofar as

$R^1$ does not represent alkylthio with 1 to 4 carbon atoms and

$R^6$ does not represent alkyl with 1 to 4 carbon atoms, for inhibiting plant growth.

**Revendication**

Utilisation de sulfonanilides de formule

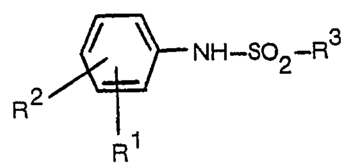

(I)

dans laquelle

$R^1$ représente un groupe phénoxy éventuellement substitué par un halogène, un groupe alkylthio en C1—C4, un groupe alkylsulfonylamino-alkylthio contenant 1 à 4 atomes de carbone dans le groupe alkylsulfonyle et 1 à 4 atomes de carbone dans le groupe alkylthio, ou le groupement de formule $-S(O)_nR^6$,

dans laquelle

$R^6$ représente un groupe alkyle en C1—C4 ou halogénoalkyle contenant 1 à 4 atomes de carbone et jusqu'à 5 atomes d'halogènes et n est égal à 1 ou 2,

$R^2$ représente l'hydrogène, le chlore ou un groupe halogénoalkyle contenant 1 à 4 atomes de carbone et jusqu'à 5 atomes d'halogènes et $R^3$ représente le groupement de formule

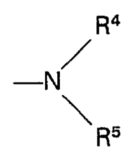

dans laquelle $R^4$ et $R^5$ représentent chacun, indépendamment l'un de l'autre, un groupe alkyle en C1—C4,

ou bien

$R^3$ représente un groupe alkyle en C1—C4 à condition que

$R^1$ ne représente pas un groupe alkylthio en C1—C4 et

$R^6$ ne représente pas un groupe alkyle en C1—C4, pour l'inhibition de la croissance des végétaux.